(19) Europäisches Patentamt
European Patent Office
Office européen des brevets

(11) Veröffentlichungsnummer: **0 005 709**
B1

(12) # EUROPÄISCHE PATENTSCHRIFT

(45) Veröffentlichungstag der Patentschrift: **09.12.81**

(21) Anmeldenummer: **79100643.0**

(22) Anmeldetag: **05.03.79**

(51) Int. Cl.³: **C 07 C 43/257,** C 07 C 69/712, A 01 N 39/02, C 07 C 125/06, C 07 C 149/14, C 07 C 147/14, C 07 C 149/36, C 07 C 147/02, C 07 D 213/64, C 07 C 121/75

(54) **Herbizid wirksame substituierte Diphenylätherderivate, diese als Wirkstoffe enthaltende herbizide Mittel und deren Verwendung.**

(30) Priorität: **15.03.78 CH 2819/78**

(43) Veröffentlichungstag der Anmeldung: **12.12.79 Patentblatt 79/25**

(45) Bekanntmachung des Hinweises auf die Patenterteilung: **09.12.81 Patentblatt 81/49**

(84) Benannte Vertragsstaaten: **BE CH DE FR GB IT NL**

(56) Entgegenhaltungen:
**DE-A-2 611 695**
**US-A-3 363 003**
**US-A-3 849 503**
**US-A-4 046 798**

(73) Patentinhaber: **CIBA-GEIGY AG, Patentabteilung Postfach, CH-4002 Basel (CH)**

(72) Erfinder: **Rohr, Otto, Dr., Kilbertweg 19, CH-4106 Therwil (CH)**
Erfinder: **Pissiotas, Georg, Dr., Breslauerstrasse 8, D-7850 Lörrach (DE)**
Erfinder: **Böhner, Beat, Dr., Hügelweg 3, CH-4102 Binningen (CH)**
Erfinder: **Szczepanski, Henry, Dr., Waldshuterstrasse 55, CH-4310 Rheinfelden (CH)**

**EP 0 005 709 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Herbizid wirksame substituierte Diphenylätherderivate, diese als Wirkstoffe enthaltende herbizide Mittel und deren Verwendung

Die vorliegende Erfindung betrifft neue, herbizid wirksame substituierte Diphenylätherderivate, Verfahren zu ihrer Herstellung, ferner herbizide Mittel, die diese neuen Wirkstoffe enthalten, sowie die Verwendung der neuen Derivate und der sie enthaltenden Mittel zur selektiven Bekämpfung von Unkräutern in Kulturpflanzenbeständen.

In der Literatur sind neben zahlreichen, in verschiedener Weise substituierten (Phenoxy)-α-phenoxy-alkancarbonsäurederivaten auch substituierte 3- und 4-(Phenoxy)-phenoxy-alkanole, ihre entsprechenden Phenylthio-Analogen, Mercaptane, Ester sowie Amine und weitere Derivate bekannt geworden (DOS 2 611 695, DOS 2 533 172, USP 3 849 503 und USP 4 046 798).

Es wurde nun gefunden, dass gewisse 3-(Phenoxy)-phenoxy-alkanole und deren Abkömmlinge diesen vorbekannten Verbindungen in mancher Hinsicht in der herbiziden Wirksamkeit überlegen sind.

Die neuen Wirkstoffe vorliegender Erfindung entsprechen der Formel I

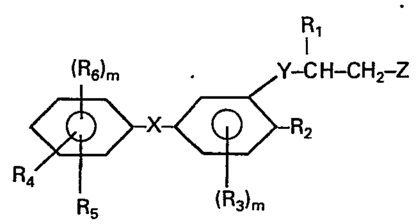

(I)

in welcher die Symbole folgende Bedeutungen haben:

$R_1$ ist Methyl,

$R_2$ ist Halogen, die Nitro- oder Cyanogruppe

$R_3$ ist Halogen, $C_1$–$C_4$-Alkyl oder die Nitrogruppe

$R_4$ und $R_5$ bedeuten unabhängig voneinander je Wasserstoff, $CF_3$, CN oder $NO_2$

$R_6$ ist Halogen

m ist jeweils eine Zahl von 0 bis 3

X und Y sind unabhängig voneinander Sauerstoff oder Schwefel

Z ist Halogen oder eine Gruppe –OH, –SH, $-OR_7$,

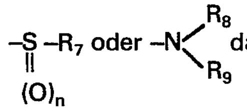 dabei $R_7$ Alkyl, Cycloalkyl, Alkenyl, Cycloalkenyl, Alkinyl, Phenyl oder Benzyl darstellt,

wobei die genannten Reste auch durch Halogen, $CF_3$, $NO_2$, CN, Alkyl, Alkylthio, Alkoxy, –OH, $NH_2$, Alkylamino, Dialkylamino und/oder Alkoxycarbonyl substituiert sein können,

$R_7$ ferner eine –CONH-Alkyl, –CO–NH-Aryl, –CO–N(Dialkyl), –CO-Aryl oder –CO-Alkyl-Gruppe darstellen kann, wobei die Alkyl- und Arylreste noch durch Halogen substituiert sein können,

$R_7$ schliesslich auch einen Rest

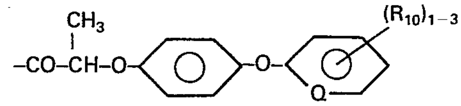

bedeuten kann, in welchem der oder die Substituenten

$R_{10}$ unabhängig voneinander Halogen, $NO_2$, CN, $CF_3$ oder Alkyl darstellen und

Q durch =CH– oder =N– verkörpert ist,

n die Zahl 0, 1 oder 2,

$R_8$ und $R_9$ sind unabhängig voneinander je Wasserstoff, $C_1$–$C_4$ Alkyl, das unsubstituiert oder durch Halogen, CN, –OH oder $C_1$–$C_4$-Alkoxy substituiert sein kann, oder

$R_8$ und $R_9$ bilden zusammen mit dem benachbarten Stickstoffatom einen 5- bis 6-gliedrigen heterocyclischen Ring, der auch noch ein Sauerstoff-, Schwefel- oder weiteres Stickstoffatom als zweites Heteroatom enthalten und durch $C_1$–$C_4$-Alkyl oder durch Phenyl substituiert sein kann, oder worin

Z für eine der Gruppen $-O-CO-O-C_2H_5$, $-O-SO_2-CH_3$ oder $-O-SO_2$-p-tolyl stehen kann, wenn gleichzeitig $R_2$ Chlor, $(R_6)_m$ 2-Chlor, $(R_3)_m$ und $R_5$ Wasserstoff, $R_4$ 4-Trifluormethyl und X und Y Sauerstoff bedeuten, oder

Z für den Rest $-O-CO-N(CH_3)OCH_3$ steht, wenn gleichzeitig $R_2$ Chlor, $(R_6)_m$ 2-Chlor, $(R_3)_m$ 4-Chlor, $R_5$ Wasserstoff, $R_4$ 4-Trifluormethyl und X und Y Sauerstoff, oder gleichzeitig $R_2$ Nitro, $(R_6)_m$ 2-Chlor, $(R_3)_m$ und $R_5$ Wasserstoff, $R_4$ 4-Trifluormethyl und X und Y Sauerstoff bedeuten.

Die erfindungsgemässen Wirkstoffe der Formel I können ausgehend von Phenoxyphenolen bzw. Phenylthio-(thio)-phenolen der Formel II

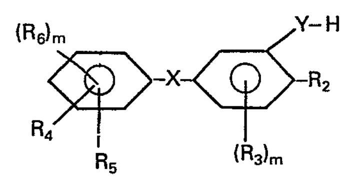

(II)

oder entsprechend Phenolaten nach vielfachen, dem Fachmann geläufigen Methoden hergestellt werden, z.B. durch Umsetzung mit Verbindungen der Formel

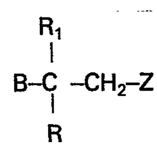

worin B Halogen oder eine Sulfoestergruppe bedeutet, wie z.B. 2-Brompropanol, gegebenenfalls in Gegenwart säurebindender Mittel und anschliessende Veresterung der so erhaltenen Propanole.

Alkohole (Z=OH) stellt man auch her durch Verseifung der entsprechenden Halogenalkylverbindungen (Z=Halogen), welche aus Alkoholen und Halogenierungsmitteln, wie Thionylchlorid erhalten wurden.

Ferner erhält man Alkohole durch Reduktion entsprechender Carbonsäureester, z.B. mit $LiAlH_4$ oder Lithiumborhydrid.

Merkaptane (Z=SH) erhält man beispielsweise aus den entsprechenden Halogenverbindungen (Z=Halogen) durch Umsetzung mit Metallhydrogensulfiden bzw. -xanthaten oder durch Umsetzung mit Thioharnstoff und anschliessender alkalischer Spaltung der entstandenen Thiuroniumsalze.

Äther und Thioäther (Z=$OR_7$ und $SR_7$) werden hergestellt durch Umsetzung der entsprechenden Halogenverbindungen (Z=Halogen) mit Alkoholen oder Merkaptanen oder durch Umsetzung der entsprechenden Alkohole bzw. Merkaptane (Z=OH oder SH) mit Halogeniden.

Ester bzw. Thioester erhält man in an sich bekannter Weise durch Umsetzung von entsprechenden Alkoholen oder Merkaptanen (Z=OH oder SH) mit Säurehalogeniden, Säureanhydriden oder Carbamoylhalogeniden in Gegenwart säurebindender Mittel.

Sulfoxide und Sulfone werden erhalten durch Oxydation der entsprechenden Thioäther, beispielsweise mit $H_2O_2$ oder Permanganat.

Amine erhält man durch Umsetzung entsprechender Halogenide (Z=Hal) mit Aminen $HNR_8R_9$.

Die Ausgangsstoffe der Formel II sind zum Teil bekannt. Noch nicht beschriebene Ausgangsstoffe der Formel II lassen sich nach bekannten Methoden herstellen, wie sie in DOS 2 643 438, 2 639 796 usw. beschrieben sind. Falls ein Substituent $R_2$ Halogen oder die Cyanogruppe ist, lässt er sich aus der entsprechenden Nitroverbindung durch Reduktion der Nitro- zur Aminogruppe, Diazotierung derselben und Ersatz der Diazogruppe durch Halogen oder –CN in bekannter Weise einführen.

Die genannten Umsetzungen werden vorzugsweise in einem gegenüber den Reaktionskomponenten inerten Lösungs- oder Verdünnungsmittel durchgeführt. Dazu eignen sich solche aus den verschiedensten Stoffklassen, wie aliphatische und aromatische, gegebenenfalls halogenierte Kohlenwasserstoffe, sowie polare inerte organische Lösungsmittel, wie Äther, Ketone, Amide etc. Als Beispiele seien Diäthyläther, Butanon, Methylenchlorid, Methyl-äthylketon, Dimethoxyäthan, Dimethylformamid und Tetrahydrofuran speziell genannt.

Als basische Säureakzeptoren (Säurebindemittel) für die Umsetzung mit Halogenverbindungen können wässerige Alkalimetallhydroxyde, wie KOH und NaOH sowie Karbonate ($K_2CO_3$, $NaHCO_3$), Alkoholate ($NaOCH_3$), aber insbesondere auch organische Basen wie Triäthylamin, Pyridin etc. verwendet werden.

Bevorzugte Verbindungen der Formel I sind jene, in denen X durch Sauerstoff verkörpert ist, also Phenoxyphenylderivate; auch Y bedeutet vorzugsweise Sauerstoff.

Unter diesen Wirkstoffen der Formel I, in denen X und Y Sauerstoff darstellen, stehen diejenigen im Vordergrund des Interesses, in denen $R_1$ die Methylgruppe und $R_3$ Halogen ist und $R_2$ entweder Halogen oder die Cyanogruppe darstellt.

Unter den weniger bedeutsamen Nitroverbindungen ($R_2$=$NO_2$) seien diejenigen der folgenden Formel erwähnt:

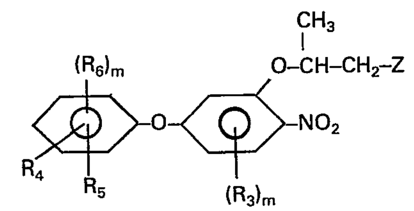

$R_3$ ist dabei Halogen und die übrigen Radikale haben die unter Formel I gegebenen Bedeutungen.

Die erfindungsgemässen Verbindungen der Formel I besitzen ein breites gutes Wirkungsspektrum insbesondere gegen mono- und dicotyle Unkräuter, werden von dicotylen Kulturpflanzen und durch verschiedene Getreidearten aber gut toleriert und können deshalb zur selektiven Bekämpfung von Unkräutern in Kulturpflanzenbeständen eingesetzt werden, und zwar sowohl in pre- als auch in post-emergenter Applikation.

Die erfindungsgemässen Verbindungen zeichnen sich gegenüber den aus dem Stand der Technik (DOS 2 611 695 und US-PS 3 849 503 und 4 046 798) bekannten Verbindungen einerseits durch bessere Herbizidwirkung und andererseits durch höhere Selektivität in Kulturpflanzenbeständen aus. Dadurch können beispielsweise schwer bekämpfbare Unkräuter mit höheren Herbizidaufwandmengen vernichtet werden, ohne dass die Kultur geschädigt wird.

Die nachfolgenden Beispiele veranschaulichen die Herstellung einiger erfindungsgemässer Wirkstoffe der Formel I. Weitere in entsprechender Weise hergestellte Wirkstoffe sind anschliessend tabellarisch aufgeführt.

Beispiel 1

1,5 g (0,39 Mol) Lithiumaluminiumhydrid werden in 100 ml absolutem Äther vorgelegt. Unter gutem Rühren werden 21,5 g (0,052 Mol) 3-(2'-Chlor-4'-trifluormethylphenoxy)-α-(6-Chlorphenoxy)-propionsäure-methylester, gelöst in 25 ml Äther, so zugetropft, dass das Reaktionsgemisch am Sieden gehalten wird. Nach der Zugabe wird 1 Std. unter Siedetemperatur nachgerührt. Nach Zugabe von 50 ml Wasser und 50 ml 10%iger Schwefelsäure wird die ätherische Phase abgetrennt und die wässrige Phase zweimal mit 100 ml Äther extrahiert. Die vereinigten Ätherextrakte werden einmal mit Wasser gewaschen und über Natriumsulfat getrocknet. Nach Abdestillieren des Lösungsmittels und Chromatographieren an Alox I erhält man 17,8 g braunes Öl, welches das Propanol der Formel

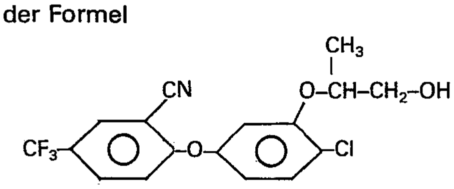

darstellt.

Analyse: C ber.: 50,37% H ber.: 3,4%
C gef.: 50,3 % H gef.: 3,5%

Beispiel 2

0,8 g Lithiumborhydrid werden in 100 ml absolutem Äther vorgelegt. Unter gutem Rühren werden 15 g 3-(2'-Chlor-4'-trifluormethylphenoxy)-α-(6-cyanophenoxy)-propionsäure-methylester, gelöst in 25 ml Äther, so zugetropft, dass das Reaktionsgemisch am Sieden gehalten wird. Nach der Zugabe wird 1 Stunde unter Siedetemperatur nachgerührt. Nach Zugabe von 8 ml Essigester, gefolgt von 4 ml 10%iger Ammoniumchloridlösung, wird abfiltriert und die ätherische Phase abgetrennt. Das Lösungsmittel wird am Rotationsverdampfer abdestilliert und das so erhaltene Öl im Kugelrohrofen destilliert. Man erhält so 7 g öliges Endprodukt der Formel

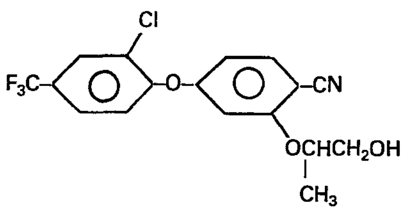

Kp: 180 °C bei 0,001 Torr.

Auf gleiche Weise können alle Derivate, die als funktionelle Gruppe CN oder $NO_2$ enthalten, reduziert werden.

Beispiel 3

3 g Natriumhydrid-Dispersion in Öl (ca. 55%) werden in 100 ml Tetrahydrofuran vorgelegt und unter gutem Rühren unter Stickstoff 19,6 g 3-(2'-Chlor-4'-trifluormethylphenoxy)-α-(6-chlorphenoxy)-propanol, gelöst in 50 ml Tetrahydrofuran, zugetropft. Es wird 1 Stunde bei ca. 35 °C ausgerührt und anschliessend bei Raumtemperatur 7 g Dimethylcarbamoylchlorid zugetropft und eine weitere Stunde ausgerührt. Nach Zugabe von Wasser wird der organische Teil abgetrennt und eingedampft. Das Öl wird im Hochvakuum destilliert: $Kp_{0,03}$: 177 °–180 °C.

Man erhält 17 g der Verbindung folgender Formel

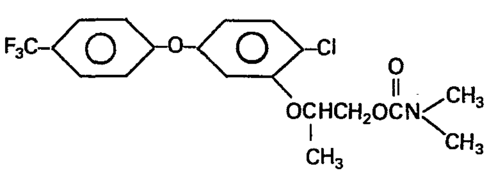

Beispiel 4

In einer Lösung von 10 g (0,03 Mol) 4-(2'-Chlor-4'-trifluormethylphenoxy)-phenol und 3,75 g (0,03 Mol) 2-Chlor-n-propylmethylsulfid in 30 ml Acetonitril werden 4,3 g (0,031 Mol) Kaliumcarbonat suspendiert und das Reaktionsgemisch 3 Stunden am Rückfluss gekocht. Nun wird eingedampft, der Rückstand mit 200 ml Äther verrührt, die Ätherlösung 3mal mit je 200 ml 20%iger Natriumcarbonatlösung gewaschen, getrocknet und eingedampft. Es werden 11 g Endprodukt erhalten, das einen Brechungsindex von $n_D^{23}$ = 1,5642 aufweist und folgende Formel hat:

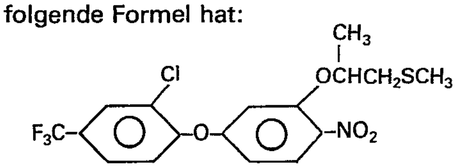

Die in den vorstehenden Beispielen beschriebenen sowie weitere erfindungsgemäss hergestellte Wirkstoffe der Formel I sind in der nachstehenden Tabelle aufgeführt, und zwar so, dass einerseits die Grundkörper der Formel I und andererseits die dazugehörigen Reste Z gesondert angegeben sind.

A.) Grundkörper

1.

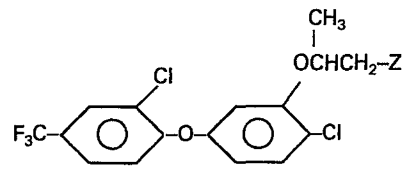

2.

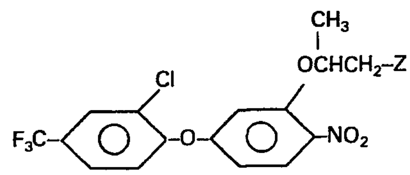

3.

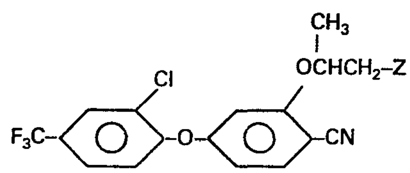

4.

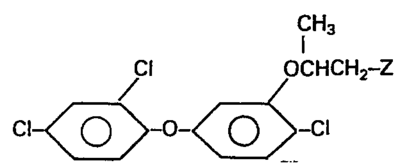

5.

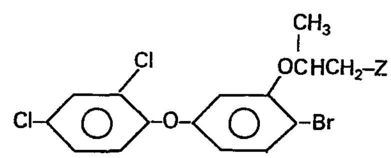

6.

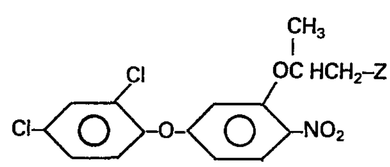

7.

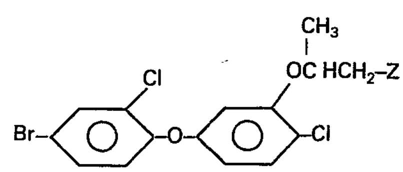

8. 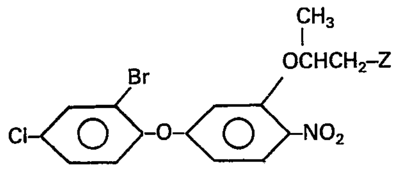

9. 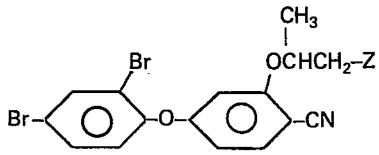

10. 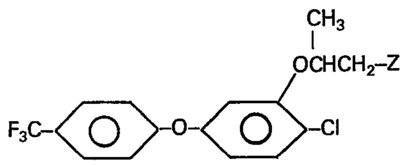

11. 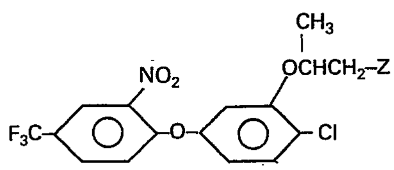

12. 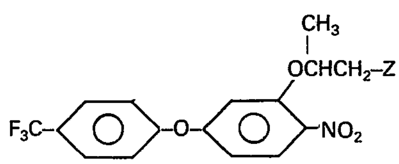

14. 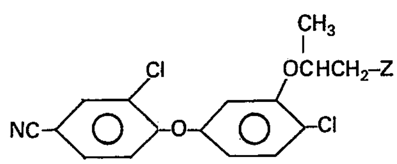

15. 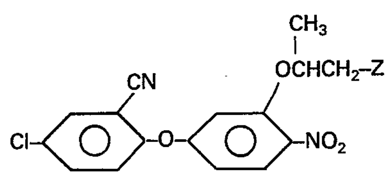

16. 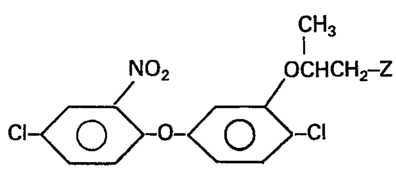

17. 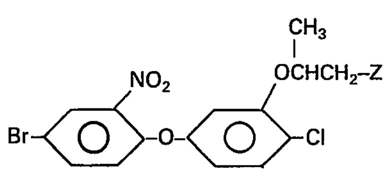

18. 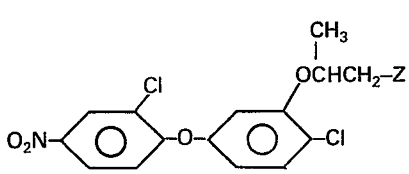

19. 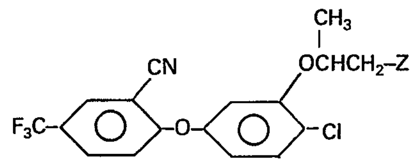

20. 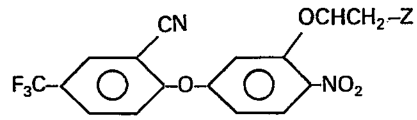

B.) Dazugehörige Reste Z

a) –OH

b) 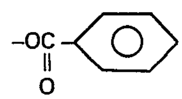

c) 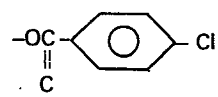

d) 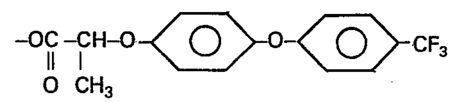

e) 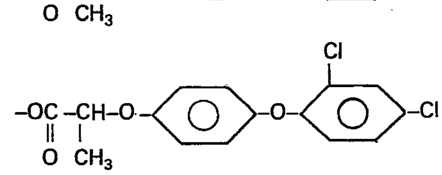

f) 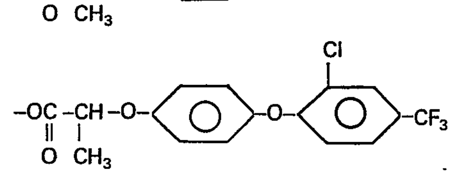

g) 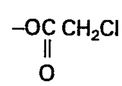

h) 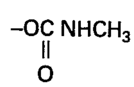

i) 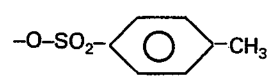

j) 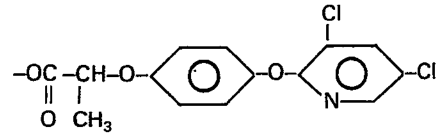

k) 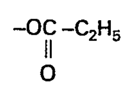

l) $-OSO_2CH_3$

m) 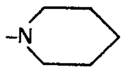

n) 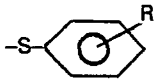 R=Hal, $NO_2$, CN, $CF_3$, Alkyl, Alkoxy

o) 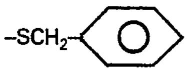

p) $-SCH_2CH{=}CH_2$

q) $-OCH_2CH{=}CH_2$

r) $-OCH_2C{\equiv}CCH$

s) $-OCH_3$

t) 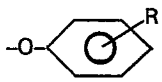 R=Hal, $NO_2$, CN, $CF_3$, Alkyl, Alkoxy

u) 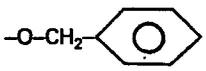

v) 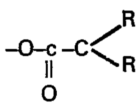 R=H, Alkyl

w) 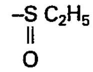

x) 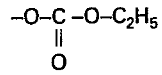

y) 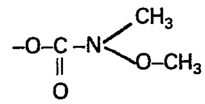

z) 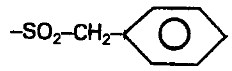

Ausgewählte Einzelverbindungen der Formel I einschliesslich der in den Beispielen beschriebenen sind nachfolgend tabellarisch zusammengestellt. Sie fallen unter folgende engere Formel:

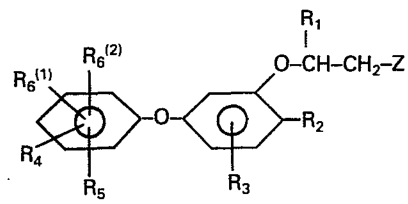

| Verbg. Nr. | $R_1$ | $R_2$ | $R_3$ | $R_4$ | $R_5$ | $R_6^{(1)}$ | $R_6^{(2)}$ | Z | physikalische Konstanten Sdp °C/Torr oder $n_D$ |
|---|---|---|---|---|---|---|---|---|---|
| 1 | $CH_3$ | Cl | H | H | H | 2–Cl | 4–Cl | –OH | $n_D^{25} = 1{,}5885$ |
| 2 | $CH_3$ | Cl | H | H | H | 2–Cl | 4–Cl | $-O-CO-NHCH_3$ | $n_D^{25} = 1{,}5733$ |
| 3 | $CH_3$ | Cl | H | H | H | 2–Cl | 4–Cl | $-O-CO-CH_2Cl$ | $n_D^{25} = 1{,}5639$ |
| 4 | $CH_3$ | Cl | H | H | H | 2–Cl | 4–Cl | –O–CO–NH– 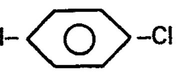 | $n_D^{25} = 1{,}5978$ |
| 5 | $CH_3$ | Cl | H | H | H | 2–Cl | 4–Cl | $-O-CO-CH_3$ | $n_D^{25} = 1{,}5662$ |
| 6 | $CH_3$ | Cl | H | H | H | 2–Cl | 4–Cl | –O–CO-Phenyl | $n_D^{25} = 1{,}5880$ |
| 7 | $CH_3$ | Cl | H | H | H | 2–Cl | 4–Cl | –O–CO–NH-Phenyl | $n_D^{25} = 1{,}5532$ |
| 8 | $CH_3$ | Cl | H | H | H | 2–Cl | 4–Cl | $-SCH_3$ | $n_D^{22} = 1{,}5970$ |
| 9 | $CH_3$ | Cl | H | H | H | 2–Cl | 4–Cl | $-SOCH_3$ | $n_D^{22} = 1{,}5930$ |
| 10 | $CH_3$ | Cl | H | 4–$CF_3$ | H | 2–Cl | H | –OH | 174–176°/0,07 |
| 11 | $CH_3$ | Cl | H | 4–$CF_3$ | H | 2–Cl | H | 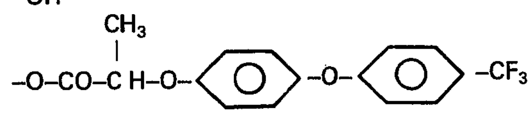 | 250°/0,001 |
| 12 | $CH_3$ | Cl | H | 4–$CF_3$ | H | 2–Cl | H | –O–CO–NH-Phenyl | 240°/0,001 |
| 13 | $CH_3$ | Cl | H | 4–$CF_3$ | H | 2–Cl | H | $-O-CO-CH_2Cl$ | 200°/0,001 |
| 14 | $CH_3$ | Cl | H | 4–$CF_3$ | H | 2–Cl | H | $-O-CO-N(CH_3)_2$ | 177–180°/0,03 |
| 15 | $CH_3$ | Cl | H | 4–$CF_3$ | H | 2–Cl | H | $-O-CO-C_2H_5$ | 200°/0,001 |
| 16 | $CH_3$ | Cl | H | 4–$CF_3$ | H | 2–Cl | H | 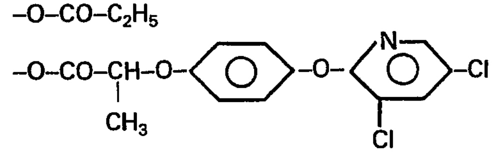 | 250°/0,001 |
| 17 | $CH_3$ | Cl | H | 4–$CF_3$ | H | 2–Cl | H | $-O-CO-NHCH_3$ | 180°/0,001 |
| 18 | $CH_3$ | Cl | H | 4–$CF_3$ | H | 2–Cl | H | –O–CO–NH– 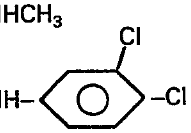 | 250°/0,001 |
| 19 | $CH_3$ | $NO_2$ | H | 4–$CF_3$ | H | 2–Cl | H | –OH | Öl |
| 20 | $CH_3$ | Cl | 4–Cl | 4–$CF_3$ | H | 2–Cl | H | –OH | 150°/0,001 |
| 21 | $CH_3$ | $NO_2$ | 4–Cl | 4–$CF_3$ | H | 2–Cl | H | –OH | 250°/0,001 |
| 22 | $CH_3$ | CN | H | 4–$CF_3$ | H | 2–Cl | H | –OH | 180°/0,001 |

| Verbdg. Nr. | $R_1$ | $R_2$ | $R_3$ | $R_4$ | $R_5$ | $R_6^{(1)}$ | $R_6^{(2)}$ | Z | physikalische Konstanten Sdp °C/Torr oder $n_D$ |
|---|---|---|---|---|---|---|---|---|---|
| 23 | $CH_3$ | Cl | H | H | H | 2–Cl | 4–Cl | 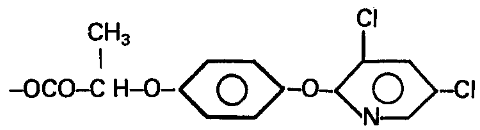 | 250°/0,001 |
| 24 | $CH_3$ | $NO_2$ | H | H | H | 2–Cl | 4–Cl | –OH | Öl |
| 25 | $CH_3$ | Cl | H | 4–$CF_3$ | 2–CN | H | H | –OH | 180°/0,001 |
| 26 | $CH_3$ | Cl | H | 4–$CF_3$ | H | 2–Cl | H | 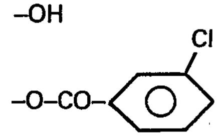 | 220°/0,001 |
| 27 | $CH_3$ | Cl | H | 4–$CF_3$ | H | 2–Cl | H | –O–CO–O–$C_2H_5$ | 170–180°/0,001 |
| 28 | $CH_3$ | Cl | H | 4–$CF_3$ | H | 2–Cl | H | –O–$SO_2$–$CH_3$ | 155°/0,001 |
| 29 | $CH_3$ | Cl | H | 4–$CF_3$ | H | 2–Cl | H | –O-Phenyl | Öl |
| 30 | $CH_3$ | Cl | H | 4–$CF_3$ | H | 2–Cl | H | 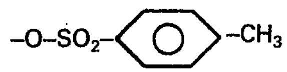 | Öl |
| 31 | $CH_3$ | Cl | H | 4–$CF_3$ | H | 2–Cl | H | 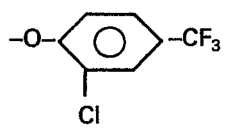 | Öl |
| 32 | $CH_3$ | Cl | 4–Cl | 4–$CF_3$ | H | 2–Cl | H | 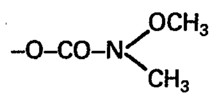 | Öl |
| 33 | $CH_3$ | Cl | H | 4–$CF_3$ | H | 2–Cl | H | –O–CO–$CH_2Cl$ | Öl |
| 34 | $CH_3$ | Cl | H | 4–$CF_3$ | H | 2–Cl | H | –S-Phenyl | Öl |
| 35 | $CH_3$ | $NO_2$ | H | 4–$CF_3$ | H | 2–Cl | H | –O–CO–$C_2H_5$ | Öl |
| 36 | $CH_3$ | $NO_2$ | H | 4–$CF_3$ | H | 2–Cl | H | 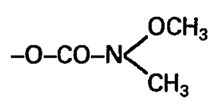 | Öl |
| 37 | $CH_3$ | $NO_2$ | H | 4–$CF_3$ | H | 2–Cl | H | –O–CO–$CH_2Cl$ | Öl |
| 38 | $CH_3$ | Cl | H | 4–$CF_3$ | H | 2–Cl | H | 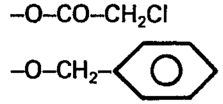 | Öl |

| Verbdg. Nr. | $R_1$ | $R_2$ | $R_3$ | $R_4$ | $R_5$ | $R_6^{(1)}$ | $R_6^{(2)}$ | Z | physikalische Konstanten Sdp °C/Torr oder $n_D$ |
|---|---|---|---|---|---|---|---|---|---|
| 39 | $CH_3$ | Cl | H | $4\text{–}CF_3$ | H | 2–Cl | H | $–SO_2–CH_3$ | Öl |
| 40 | $CH_3$ | $NO_2$ | H | $4\text{–}CF_3$ | H | 2–Cl | H | $–S–C_2H_5$ | Öl |
| 41 | $CH_3$ | Cl | H | $4\text{–}CF_3$ | H | 2–Cl | H | $–SCH_3$ | $n_D^{23} = 1{,}5525$ |
| 42 | $CH_3$ | $NO_2$ | H | $4\text{–}CF_3$ | H | 2–Cl | H | $–O–CO–C_2H_5$ | Öl |
| 43 | $CH_3$ | Cl | H | $4\text{–}CF_3$ | H | 2–Cl | H | $–OCH_3$ | Öl |
| 44 | $CH_3$ | $NO_2$ | H | $4\text{–}CF_3$ | H | 2–Cl | H | $–SCH_3$ | Öl |
| 45 | $CH_3$ | Cl | H | $4\text{–}CF_3$ | H | 2–Cl | H | Cl | 148–149°/0,3 |
| 46 | $CH_3$ | Cl | H | $4\text{–}CF_3$ | H | 2–Cl | H | $–O–CH_2–CH{=}CH_2$ | Öl |
| 47 | $CH_3$ | Cl | H | $4\text{–}CF_3$ | H | 2–Cl | H | $–O–CH_2–C{\equiv}CH$ | Öl |

Verbindung Nr. 48:

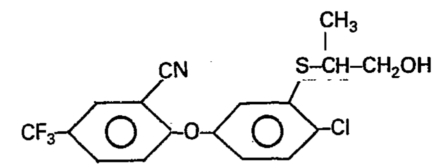

Öl

Die Erfindung betrifft auch herbizide Mittel, welche einen neuen Wirkstoff der Formel I enthalten, sowie Verfahren zur pre- und post-emergenten Unkrautbekämpfung.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen und/oder Verteilungsmitteln, gegebenenfalls unter Zusatz von gegenüber den Wirkstoffen inerten Antischaum-, Netz-, Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsgranulate, Imprägnierungsgranulate und Homogengranulate; |
| in Wasser dispergierbare Wirkstoffkonzentrate: | Spritzpulver (wettable powder), Pasten, Emulsionen, Emulsionskonzentrate; |
| flüssige Aufarbeitungsformen: | Lösungen. |

Die Wirkstoffkonzentrationen betragen in den erfindungsgemässen Mitteln 1 bis 80 Gew.-% und können vor der Anwendung auch auf niedrige Konzentrationen, wie etwa 0,05 bis 1% verdünnt werden. Die Aufwandmengen betragen in der Regel 0,1 bis 10 kg Wirkstoff pro Hektar, vorzugsweise 0,25 bis 4 kg/ha.

Die Herstellung erfindungsgemässer Mittel wird durch die folgenden Formulierungsbeispiele veranschaulicht. Teile bedeuten darin Gewichtsteile.

Granulat: Zur Herstellung eines 5%igen Granulates werden die folgenden Stoffe verwendet:

5 Teile Wirkstoff,
0,25 Teile Epichlorhydrin,
0,25 Teile Cetylpolyäthylenglykoläther mit 8 Mol Äthylenoxid,
3,50 Teile Polyäthylenglykol,
91 Teile Kaolin (Korngrösse 0,3 bis 0,8 mm).

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

Spritzpulver: Zur Herstellung eines a) 50%igen, b) 25%igen und c) 10%igen Spritzpulvers werden folgende Bestandteile verwendet:

a) 50 Teile Wirkstoff gemäss Beispiel 1,
5 Teile Natriumdibutylnaphthylsulfonat,
3 Teile Naphthalinsulfonsäuren-Phenolsulfonsäuren-Formaldehyd-Kondensat 3:2:1,
20 Teile Kaolin,
22 Teile Champagne-Kreide;

b) 25 Teile des obigen Wirkstoffes,
5 Teile Oleylmethyltaurid-Natrium-Salz
2,5 Teile Naphthalinsulfonsäure-Formaldehyd-Kondensat,
0,5 Teile Carboxymethylcellulose,
5 Teile neutrales Kalium-Aluminium-Silikat,
62 Teile Kaolin;

c) 10 Teile des obigen Wirkstoffes,
3 Teile Gemisch der Natriumsalze von gesättigten Fettalkoholen,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Ungräsern in Kulturpflanzen verwendet.

Paste: Zur Herstellung einer 45%igen Paste werden folgende Stoffe verwendet:

45 Teile Wirkstoff,
5 Teile Natriumaluminiumsilikat,
14 Teile Cetylpolyäthylenglykoläther mit 8 Mol Äthylenoxid,
1 Teil Oleylpolyäthylenglykoläther mit 5 Mol Äthylenoxid,
2 Teile Spindelöl,
23 Teile Wasser,
10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat: Zur Herstellung eines 25%igen Emulsionskonzentrates werden

25 Teile Wirkstoff,
10 Teile Mischung von Nonylphenolpolyoxyäthylen und Calcium-dedecylbenzol-sulfonat,
10 Teile Cyclohexanon,
55 Teile Xylol.

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffes kann auch eine andere von der Formel I umfasste Verbindung verwendet werden.

Erfindungsgemässe Mittel, die als aktive Komponente mindestens einen Wirkstoff der Formel I enthalten, eignen sich besonders zur selektiven Bekämpfung monocotyler und dicotyler Unkräuter in pre- und post-emergenter Anwendung in dicotylen Kulturpflanzenbeständen, wie z.B. Soja, Baumwolle, Zuckerrübe, Leguminosen, Klee, Luzerne, Melone, Gurke, Tabak usw, aber auch in monocotylen Kulturen von Getreide, Mais und Reis.

Zum Nachweis der Brauchbarkeit als Herbizide (pre- und post-emergent) dienen folgende Testmethoden:

Pre-emergente Herbizid-Wirkung
(Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Spritzpulver oder Emulsionskonzentrat, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22–25 °C und 50–70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)

Als Versuchspflanzen dienen:

| | |
|---|---|
| hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomoea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

Post-emergente Herbizid-Wirkung
(Kontaktherbizid)

Eine grössere Anzahl (mindestens 7) Unkräuter und Kulturpflanzen, sowohl monocotyle wie dicotyle, wurden nach dem Auflaufen (im 4- bis 6-Blattstadium) mit einer wässrigen Wirkstoffdispersion in Dosierungen von 0,25; 0,5; 1, 2 und 4 kg Wirksubstanz pro Hektar auf die Pflanzen gespritzt und diese bei 24°–26 °C und 45–60% rel. Luftfeuchtigkeit gehalten. 15 Tage nach Behandlung wird der Versuch ausgewertet und das Ergebnis wie im pre-emergent-Versuch nach derselben Notenskala bonitiert.

In einem Vergleichsversuch wurden 4 erfindungsgemässe Wirkstoffe (Nrn. 10, 12, 13, 16) mit zwei ähnlich substituierten 4-Phenoxy-phenoxydiphenylätherderivaten der DOS 2 611 695 in der post-emergenten Wirkung auf mono- und dicotyle Unkräuter vergleichend geprüft.

Die Methode und Bewertung entsprach dem oben beschriebenen Kontaktherbizidtest bei einer Aufwandmenge von 4 kg Wirksubstanz pro Hektar.

Als Vergleichssubstanzen der DOS 2 611 695 dienten

A: 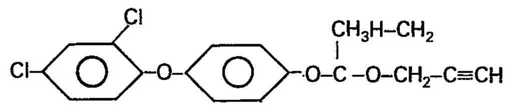

B: 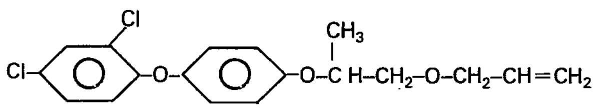

Resultate

| Unkräuter | erfindungsgemässe Verbindungen | | | | Stand der Technik (DOS 2 611 695) | |
|---|---|---|---|---|---|---|
| Nrn. | 10 | 12 | 13 | 16 | A | B |
| Avena fatua | 1 | 1 | 1 | 1 | 7 | 7 |
| Setaria | 1 | 1 | 2 | 1 | 8 | 4 |
| Lolium | 2 | 1 | 1 | 2 | 7 | 6 |
| Solanum | 1 | 1 | 1 | 1 | 8 | 9 |
| Sinapis | 1 | 1 | 1 | 1 | 8 | 8 |
| Stellaria | 1 | 1 | 1 | 1 | 8 | 7 |

**Patentansprüche**

1. Neue herbizid wirksame substituierte Diphenylätherderivate von der Formel I

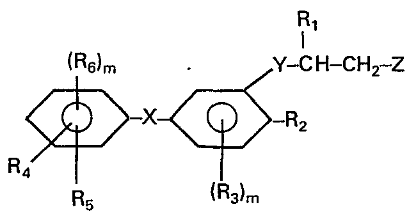

(I)

in welcher

$R_1$ eine Methylgruppe,

$R_2$ Halogen, die Nitro- oder Cyanogruppe,

$R_3$ Halogen, $C_1$–$C_4$-Alkyl oder die Nitrogruppe,

$R_4$ und $R_5$ unabhängig voneinander je Wasserstoff, $-CF_3$, $-CN$ oder $-NO_2$

$R_6$ Halogen,

m jeweils eine Zahl von 0 bis 3,

X und Y unabhängig voneinander ein Sauerstoff- oder Schwefelatom und

Z Halogen oder eine Gruppe $-OH$, $-SH$, $-OR_7$,

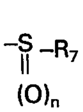 oder 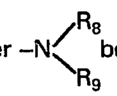 bedeuten, in welch letzteren

Gruppen die Reste folgende Bedeutungen haben:

$R_7$ Alkyl, Cycloalkyl, Alkenyl, Cycloalkenyl, Alkinyl, Phenyl oder Benzyl, welche Reste noch durch Halogen, $CF_3$, $NO_2$, CN, Alkyl, Alkoxy, Alkylthio, $-OH$, $-NH_2$ Alkylamino, Dialkylamino und/oder Alkoxycarbonyl substituiert sein können,

$R_7$ ferner eine –CO–NH-Alkyl-, –CO–NH-Aryl-, –CO–N-(Dialkyl)-, –CO-Aryl- oder –CO-Alkyl-Gruppe, wobei die Alkyl- und Arylreste noch durch Halogen substituiert sein können,

$R_7$ schliesslich auch ein Rest

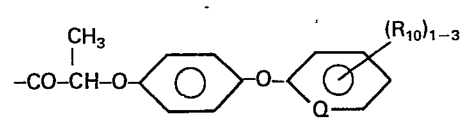

in welchem der oder die Substituenten $R_{10}$ unabhängig voneinander Halogen, $NO_2$, CN, $CF_3$ oder Alkyl darstellen und Q durch $-CH=$ oder $-N=$ verkörpert ist,

n die Zahl 0, 1 oder 2,

$R_8$ und $R_9$ unabhängig voneinander je Wasserstoff, $C_1$–$C_4$-Alkyl, das noch durch Halogen, CN, OH oder $C_1$–$C_4$-Alkoxy substituiert sein kann, oder

$R_8$ und $R_9$ bilden zusammen mit dem benachbarten Stickstoffatom einen 5- bis 6-gliedrigen heterocyclischen Ring, der noch ein Sauerstoff-, Schwefel- oder weiteres Stickstoffatom als zweites Heteroatom enthalten und durch $C_1$–$C_4$-Alkyl oder durch Phenyl substituiert sein kann, oder worin

Z für eine der Gruppen $-O-CO-O-C_2H_5$, $-O-SO_2-CH_3$ oder $-O-SO_2$-p-tolyl stehen kann, wenn gleichzeitig $R_2$ Chlor, $(R_6)_m$ 2-Chlor, $(R_3)_m$ und $R_5$ Wasserstoff, $R_4$ 4-Trifluormethyl und X und Y Sauerstoff bedeuten, oder

Z für den Rest $-O-CO-N(CH_3)OCH_3$, steht, wenn gleichzeitig $R_2$ Chlor, $(R_6)_m$ 2-Chlor, $(R_3)_m$ 4-Chlor, $R_5$ Wasserstoff, $R_4$ 4-Trifluormethyl und X und Y Sauerstoff, oder gleichzeitig $R_2$ Nitro, $(R_6)_m$ 2-Chlor, $(R_3)_m$ und $R_5$ Wasserstoff, $R_4$ 4-Trifluormethyl und X und Y Sauerstoff bedeuten.

2. Diphenylätherderivate gemäss Anspruch 1, von der Formel

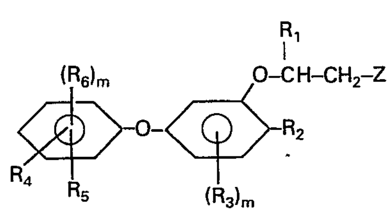

in welcher $R_2$ Halogen oder die Cyanogruppe, $R_3$ Halogen ist und die übrigen Symbole wie unter Anspruch 1 definiert sind.

3. Diphenylätherderivate gemäss Anspruch 1, von der Formel

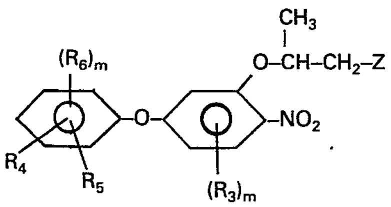

in welcher $R_3$ Halogen ist und die übrigen Symbole dieselben Bedeutungen haben wie im Anspruch 1.

4. Diphenylätherderivate gemäss Anspruch 1, von der Formel

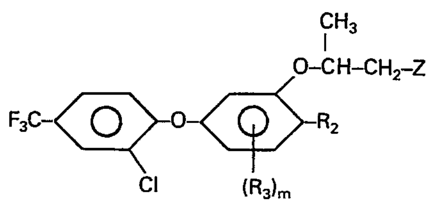

worin $R_2$ Halogen oder die Cyanogruppe ist, $R_3$ Halogen bedeutet und m und Z wie unter Anspruch 1 definiert sind.

5. Diphenylätherderivate gemäss Anspruch 1, von der Formel

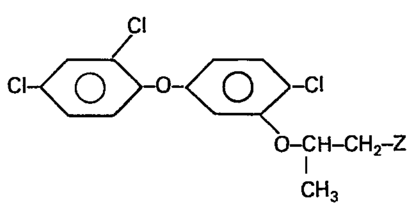

worin Z wie unter Formel I des Anspruchs 1 definiert ist.

6. Diphenylätherderivate gemäss Anspruch 1, von der Formel

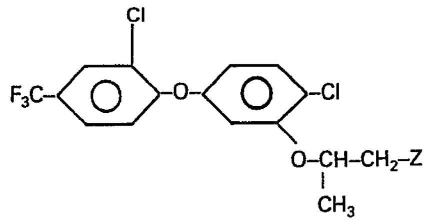

worin Z wie unter Formel I des Anspruchs 1 definiert ist.

7. Diphenylätherderivate gemäss Anspruch 1, von der Formel

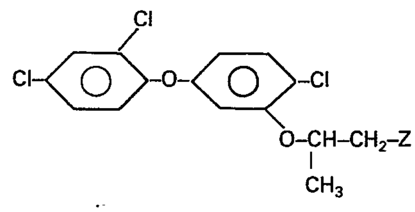

worin Z wie unter Formel I des Anspruchs 1 definiert ist.

8. Diphenylätherderivate gemäss Anspruch 1, von der Formel

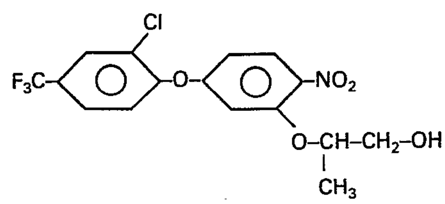

worin Z wie unter Formel I des Anspruchs 1 definiert ist.

9. Die Verbindung 2-[3'-(2''-Chlor-4''-trifluormethylphenoxy)-6'-chlorphenoxy]-propanol der Formel

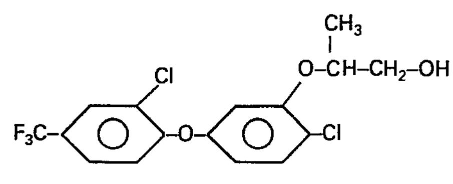

10. Die Verbindung der Formel

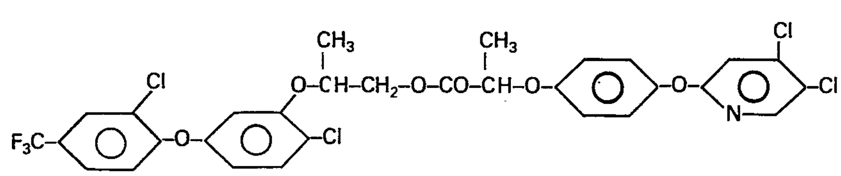

11. Die Verbindung 2-[3'-(2''-Cyano-4''-trifluormethylphenoxy)-6'-chlorphenoxy]-propanol der Formel

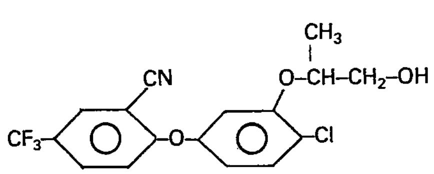

12. Die Verbindung 2-[3'-(2''Chlor-4'-trifluormethylphenoxy)-6'-cyanophenoxy]-propanol der Formel

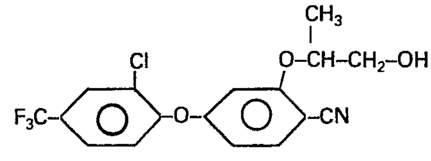

13. Die Verbindung 2-[3'-(2''-Chlor-4''-trifluormethylphenoxy)-6'-nitro-phenoxy]-propanol der Formel

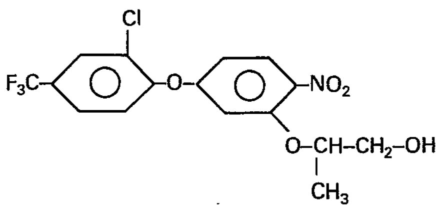

14. Die Verbindung 2-[3'-(2'',4''-Dichlorphenoxy)-6'-nitrophenoxy]-propanol der Formel

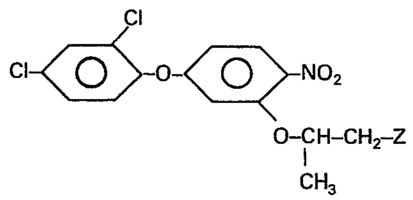

15. Die Verbindung 2-[3'-(2''-Chlor-4''-trifluormethylphenoxy)-6'-chlorphenoxy]-propanthiolmethyläther der Formel

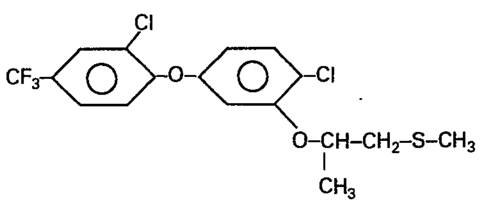

16. Herbizides Mittel, dadurch gekennzeichnet, dass es als wirksame Komponente ein Diphenylätherderivat der Formel I des Anspruchs 1 enthält.

17. Herbizides Mittel gemäss Anspruch 16, dadurch gekennzeichnet, dass es als wirksame Komponente ein Diphenylätherderivat gemäss einem der Ansprüche 2 bis 15 enthält.

**Revendications**

1. Nouveaux dérivés d'éther diphénylique substitués ayant une activité herbicide, de formule I

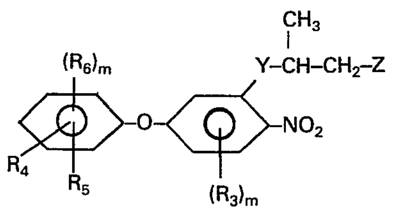

où

$R_1$ est un méthyle,

$R_2$ est un halogène, le groupe nitro ou cyano,

$R_3$ est un halogène, un alcoyle en $C_1$ à $C_4$ ou le groupe nitro,

$R_4$ et $R_5$ représentent indépendamment l'un de l'autre chacun un hydrogène, $CF_3$, CN ou $NO_2$,

$R_6$ est un halogène,

m est toujours un nombre de 0 à 3,

X et Y représentent indépendamment l'un de l'autre un oxygène ou un soufre,

Z est un halogène ou un groupe –OH, –SH,

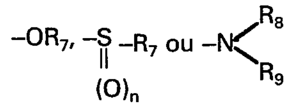

où $R_7$ représente un alcoyle, un cycloalcoyle, un alcényle, un cycloalcényle, un alcynyle, un phényle ou un benzyle, les radicaux mentionnés pouvant également être substitués par un halogène, $CF_3$, $NO_2$, CN, un alcoyle, un alcoylthio, un alcoxy, –OH, $NH_2$, un alcoylamino, un dicaloylamino et/ou un alcoxycarbonyle,

$R_7$ peut représenter en outre un groupe –CONH-alcoyle, –CO–NH-aryle, –CO–N(dialcoyle), –CO-aryle ou –CO-alcoyle, les radicaux alcoyle et aryle pouvant encore être substitués par un halogène,

$R_7$ pouvant enfin représenter également un radical

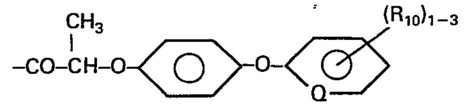

où le ou les substituant(s) $R_{10}$ représente(nt) indépendamment l'un de l'autre un halogène, $NO_2$, CN, $CF_3$ ou un alcoyle et

Q représente =CH– ou =N–,

n le nombre 0, 1 ou 2,

$R_8$ et $R_9$ représentent indépendamment l'un de l'autre chacun un hydrogène, un alcoyle en $C_1$ à $C_4$, non substitué ou pouvant être substitué par un halogène, CN, –OH ou un alcoxy en $C_1$ à $C_4$, ou

$R_8$ et $R_9$ forment ensemble avec l'atome d'azote voisin un noyau hétérocyclique à 5 à 6 chaînons, qui peut également contenir comme second hétéroatome un atome d'oxygène, de soufre ou un autre atome d'azote et être substitué par un alcoyle en $C_1$ à $C_4$ ou un phényle, ou bien où

Z peut représenter l'un des groupes –O–CO–O–$C_2H_5$, –O–$SO_2$–$CH_3$ ou –O–$SO_2$-p-tolyle, lorsque simultanément $R_2$ représente un chlore, $(R_6)_m$ un 2-chloro-, $(R_3)_m$ et $R_5$ un hydrogène, $R_4$ un 4-trifluorométhyle et X et Y un hydrogène, ou

Z représente un radical –O–CO–N($CH_3$)$OCH_3$, lorsque simultanément $R_2$ représente un chlore, $(R_6)_m$ un 2-chloro, $(R_3)_m$ un 4-chloro, $R_5$ un hydrogène, $R_4$ un 4-trifluorométhyle et X et Y un oxygène, ou bien où simultanément $R_2$ représente un nitro, $(R_6)_m$ un 2-chloro, $(R_3)_m$ et $R_5$ un hydrogène, $R_4$ un 4-trifluorométhyle et X et Y un oxygène.

2. Dérivés d'éther diphénylique selon la revendication 1, de formule

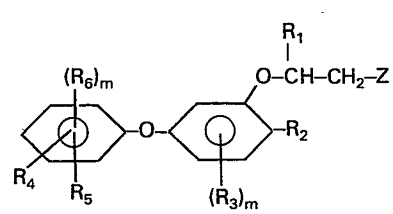

où $R_2$ représente un halogène ou le groupe cyano, $R_3$ un halogène et où les autres symboles sont définis comme dans la revendication 1.

3. Dérivés d'éther diphénylique selon la revendication 1 de formule

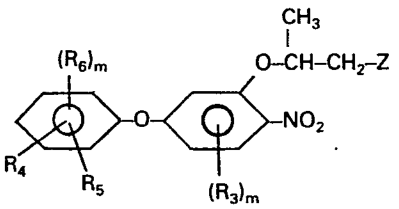

où $R_3$ est un halogène et où les autres symboles ont les mêmes significations que dans la revendication 1.

4. Dérivés d'éther diphénylique selon la revendication 1, de formule

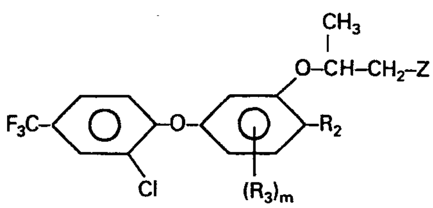

où $R_2$ est un halogène ou le groupe cyano, $R_3$ représente un halogène et n et Z sont définis comme pour la revendication 1.

5. Dérivés d'éther diphénylique selon la revendication 1, de formule

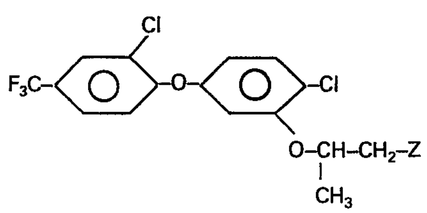

où Z est défini comme pour la formule I de la revendication 1.

6. Dérivés d'éther diphénylique selon la revendication 1, de formule

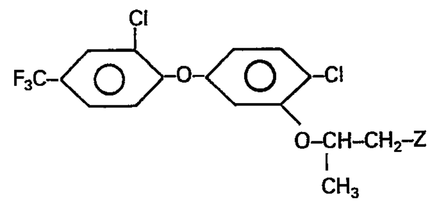

où Z est défini comme pour la formule I de la revendication 1.

7. Dérivés d'éther diphénylique selon la revendication 1, de formule

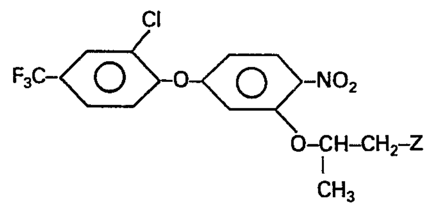

où Z est défini comme pour la formule I de la revendication 1.

8. Dérivés d'éther diphénylique selon la revendication 1, de formule

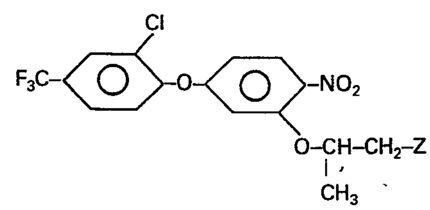

où Z est défini comme pour la formule I de la revendication 1.

9. Le composé 2-[3'-(2''-Chloro-4''-trifluorométhylphénoxy)-6'-chlorophénoxy]-propanol de formule

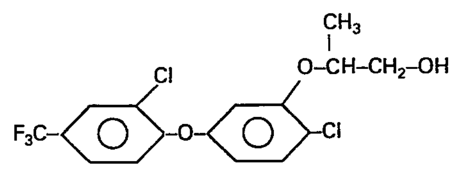

10. Le composé de formule

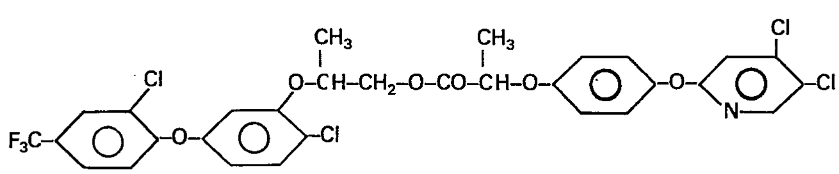

11. Le composé 2-[3'-(2''-Cyano-4''-trifluorométhylphénoxy)-6'-chlorophénoxy]-propanol de formule

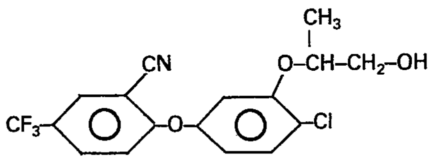

12. Le composé 2-[3'-(2''-Chloro-4''-trifluorométhylphénoxy)-6'-cyanophénoxy]-propanol de formule

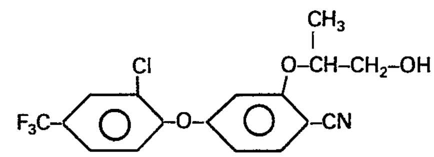

13. Le composé 2-[3'-(2''-Chloro-4''-trifluoro-méthylphénoxy)-6'-nitro-phénoxy]-propanol de formule

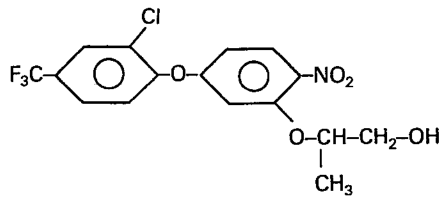

14. Le composé 2-[3'-(2'',4''-Dichlorophénoxy)-6'-nitro-phénoxy]-propanol de formule

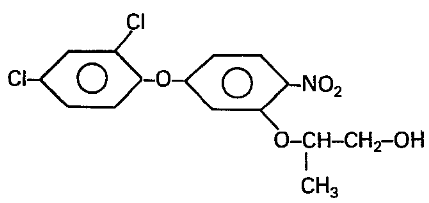

15. Le composé 2-[3'-(2''-Chloro-4''-trifluoro-méthylphénoxy)-6'-chlorophénoxy]-propanthiol-méthyléther de formule

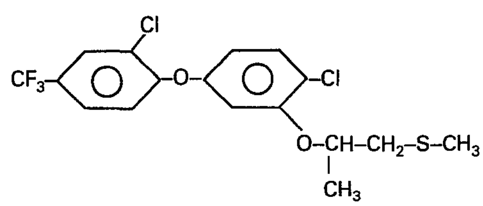

16. Agent herbicide, caractérisé en ce qu'il contient comme composant actif un dérivé d'éther diphénylique de formule I de la revendication 1.

17. Agent herbicide selon la revendication 16, caractérisé en ce qu'il contient comme composant actif un dérivé d'éther diphénylique selon l'une des revendications 2 à 15.

**Claims**

1. A herbicidally active substituted diphenyl ether derivative of the formula I

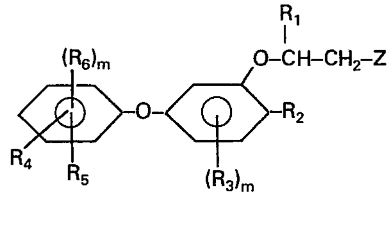

wherein

$R_1$ is a methyl group,

$R_2$ is halogen, the nitro or cyano group,

$R_3$ is halogen, $C_1$–$C_4$ alkyl or the nitro group,

$R_4$ and $R_5$, each independently of the other, are hydrogen, $CF_3$, CN or $NO_2$,

$R_6$ is halogen,

m is an integer from 0 to 3,

X and Y, each independently of the other, are an oxygen or sulfur atom,

Z is halogen or a –OH, –SH, $-OR_7$, $-S-R_7$ or
$(O)_n$

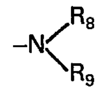 group,

wherein $R_7$ is alkyl, cycloalkyl, alkenyl, cycloalkenyl, alkynyl, phenyl or benzyl, which radicals can also be substituted by halogen, $CF_3$, $NO_2$, CN, alkyl, alkoxy, alkylthio, –OH, $NH_2$, alkylamino, dialkylamino, and/or alkoxycarbonyl, and $R_7$ can also be a –CONH-alkyl, –CO–NH-aryl, –CO–N(dialkyl), –CO-aryl or –CO-alkyl group, the alkyl and aryl moieties of which can additionally be substituted by halogen, and $R_7$ can finally also be a radical

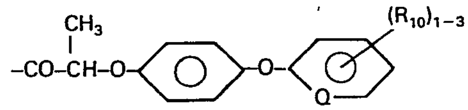

in which the recurring or non-recurring substituent $R_{10}$ can be the same or different and is halogen, $NO_2$, CN, $CF_3$ or alkyl, Q is =CH– or =N– and n is 0, 1 or 2, and $R_8$ and $R_9$, each independently of the other, are hydrogen, $C_1$–$C_4$ alkyl which can be unsubstituted or substituted by halogen, CN, –OH or $C_1$–$C_4$-alkoxy, or $R_8$ and $R_9$, together with the adjacent nitrogen atom, form a 5- to 6-membered heterocyclic ring which can additionally contain an oxygen, sulfur or further nitrogen atom as second heteroatom and can be substituted by $C_1$–$C_4$ alkyl or phenyl, or wherein

Z is one of the groups $-O-CO-O-C_2H_5$, $-O-SO_2-CH_3$ or $-O-SO_2$-p-tolyl, if at the same time $R_2$ is chlorine, $(R_6)_m$ is 2-chlorine, $(R_3)_m$ and $R_5$ are hydrogen, $R_4$ is 4-trifluoromethyl and X and Y are oxygen, or

Z is the radical $-O-CO-N(CH_3)OCH_3$, if at the same time $R_2$ is chlorine, $(R_6)_m$ is 2-chlorine, $(R_3)_m$ is 4-chlorine, $R_5$ is hydrogen, $R_4$ is 4-trifluoromethyl, and X and Y are oxygen, or at the same time $R_2$ is nitro, $(R_6)_m$ is 2-chlorine, $(R_3)_m$ and $R_5$ are hydrogen, $R_4$ is 4-trifluoromethyl and X and Y are oxygen.

2. A diphenyl ether derivative according to claim 1 of the formula

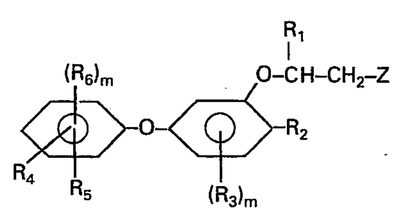

wherein $R_2$ is halogen or the cyano group, $R_3$ is halogen, and the other symbols are as defined in claim 1.

3. A diphenyl ether derivative according to claim 1 of the formula

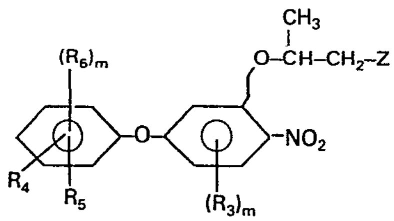

wherein $R_3$ is halogen and the other symbols are as defined in claim 1.

4. A diphenyl ether derivative according to claim 1 of the formula

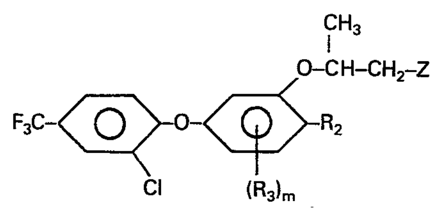

wherein $R_2$ is halogen or the cyano group, $R_3$ is halogen, and m and Z are as defined in claim 1.

5. A diphenyl ether derivative according to claim 1 of the formula

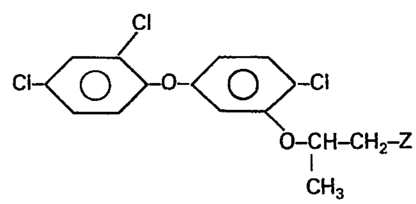

wherein Z is as defined for formula I in claim 1.

6. A diphenyl ether derivative according to claim 1 of the formula

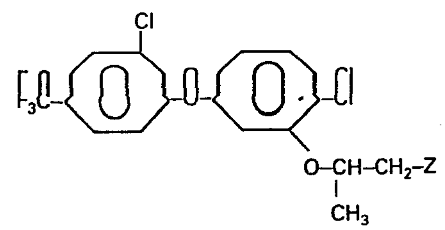

wherein Z is as defined for formula I in claim 1.

7. A diphenyl ether derivative according to claim 1 of the formula

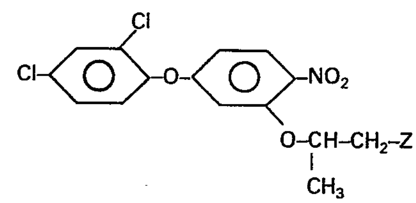

wherein Z is as defined for formula I in claim 1.

8. A diphenyl ether derivative according to claim 1 of the formula

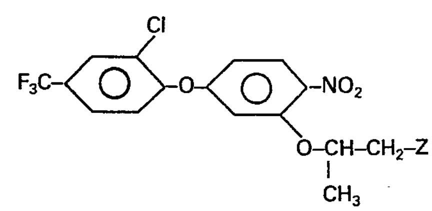

wherein Z is as defined for formula I in claim 1.

9. 2-[3'-(2''-Chloro-4''-trifluoromethylphenoxy)-6'-chlorophenoxy]-propanol of the formula

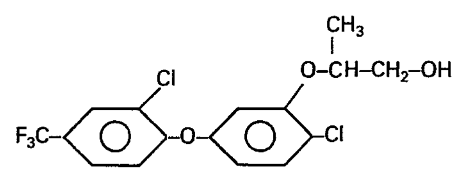

10. The compound of the formula

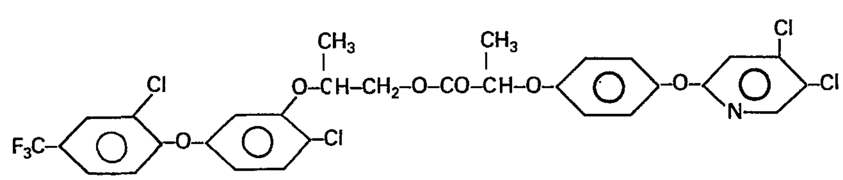

11. 2-[3'-(2''-Cyano-4''-trifluoromethylphenoxy)-6'-chlorophenoxy]-propanol of the formula

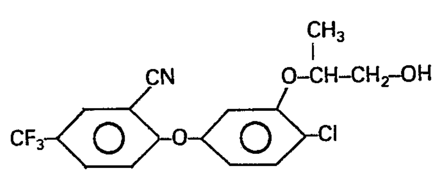

12. 2-[3'-(2''-Chloro-4''-trifluoromethylphenoxy)-6'-cyanophenoxy]-propanol of the formula

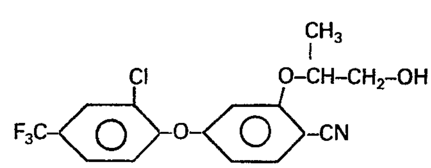

13. 2-[3'-(2''-Chloro-4''-trifluoromethylphenoxy)-6'-nitrophenoxy]-propanol of the formula

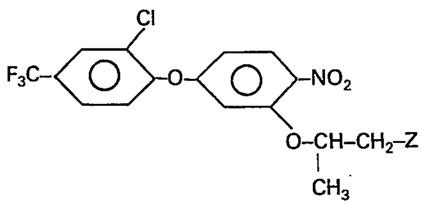

14. 2-[3'-(2'',4''-Dichlorophenoxy)-6'-nitrophenoxy]-propanol of the formula

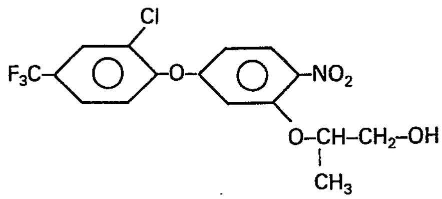

15. 2-[3'-(2''-Chloro-4''-trifluoromethylphenoxy)-6'-chlorophenoxy]-propanethiol methyl ether of the formula

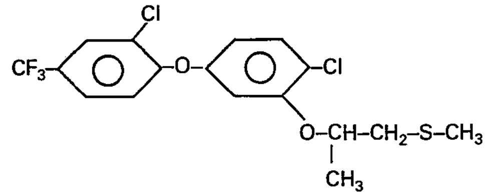

16. A herbicidal composition which contains a diphenyl ether derivative of the formula I of claim 1 as active component.

17. A herbicidal composition according to claim 16 which contains a diphenyl ether derivative according to anyone of claims 2 to 15 as active component.